(19) Europäisches Patentamt
European Patent Office
Office européen des brevets

(11) Veröffentlichungsnummer : **0 072 954**
**B1**

(12) # EUROPÄISCHE PATENTSCHRIFT

(45) Veröffentlichungstag der Patentschrift : **30.12.86**

(51) Int. Cl.[4] : **C 07 D209/44**, C 07 D403/12, A 61 K 31/40

(21) Anmeldenummer : **82107151.1**

(22) Anmeldetag : **07.08.82**

(54) **Isoindolin-2-yl-amino-imidazoline und Isoindolin-2-yl-guanidine, Verfahren zu ihrer Herstellung und ihre Verwendung als Arzneimittel.**

(30) Priorität : **22.08.81 DE 3133302**

(43) Veröffentlichungstag der Anmeldung : **02.03.83 Patentblatt 83/09**

(45) Bekanntmachung des Hinweises auf die Patenterteilung : **30.12.86 Patentblatt 86/52**

(84) Benannte Vertragsstaaten : **AT BE CH DE FR GB IT LI NL SE**

(56) Entgegenhaltungen :
**EP-B- 0 000 151**
**DE-A- 2 816 627**
**DE-A- 2 905 501**
**GB-A- 1 014 658**
**CHEMICAL ABSTRACTS, Band 96, Nr. 7, 15. Februar 1982, Seite 606, Nr. 52154e, Columbus, Ohio, USA N. VISWANATHAN et al.: "Synthesis and hypotensive activity of some aminoguanidines"**
**Die Akte enthält technische Angaben, die nach dem Eingang der Anmeldung eingereicht wurden und die nicht in dieser Patentschrift enthalten sind.**

(73) Patentinhaber : **Beiersdorf Aktiengesellschaft**
**Unnastrasse 48**
**D-2000 Hamburg 20 (DE)**

(72) Erfinder : **Cohnen, Erich, Dr. Dipl.-Chem.**
**Heilwigstrasse 25**
**D-2000 Hamburg 20 (DE)**
Erfinder : **Armah, Ben, Dr.**
**Milcher Strasse 9d**
**D-2000 Hamburg 52 (DE)**

**EP 0 072 954 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Gegenstand der Erfindung sind neue Isoindolin-2-yl-guanidine und -aminoimidazoline der allgemeinen Formel I

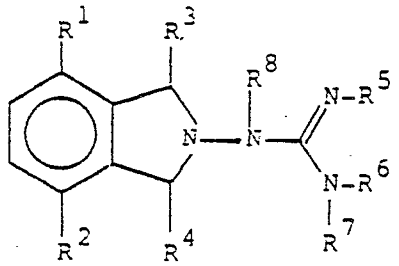

(I)

in der

$R^1$ und $R^2$, die gleich oder verschieden sein können, Wasserstoff oder Halogen, bedeuten, wobei mindestens einer der Substituenten $R^1$, $R^2$ Halogen ist,

$R^3$ und $R^4$, die gleich oder verschieden sein können, Wasserstoff oder Alkyl mit 1 bis 4 Kohlenstoffatomen.

$R^5$, $R^6$, $R^7$ und $R^8$, die gleich oder verschieden sein können, Wasserstoff oder Alkyl mit 1 bis 4 Kohlenstoffatomen oder

$R^5$ und $R^6$ zusammen die Äthylengruppe bedeuten,

sowie deren Salze, ausgenommen 4-Chlor-2-(2-imidazolin-2-ylamino)-isoindolin, Verfahren zu ihrer Herstellung, diese Verbindungen enthaltende Arzneimittel und deren Verwendung.

Die vorstehend genannte Verbindung ist aus der DE-A-29 05 501 bekannt.

Ein bevorzugter Rest $R^1$ oder $R^2$ ist Chlor. Die Reste $R^3$, $R^4$, $R^5$, $R^6$, $R^7$ und $R^8$ sind insbesondere Methyl oder Äthyl. Vorzugsweise ist $R^8$ auch Wasserstoff.

Die erfindungsgemäßen Verbindungen, insbesondere die Imidazoline, tragen vorzugsweise in der 1,3 und/oder 4,7-Stellung des Isoindolins jeweils einen oder insbesondere zwei Substituenten $R^3$ und $R^4$ und/oder $R^1$ und $R^2$.

Beispiele für Verbindungen der allgemeinen Formel I mit einem therapeutischen Effekt sind nachstehend aufgeführt :

1-Methyl-3-(4-chlor-isoindolin-2-yl)-guanidin
(3-Methyl-4-chlor-isoindolin-2-yl)-guanidin
4,7-Dibrom-2-(2-imidazolin-2-yl-amino)-isoindolin
(4,7-Dibrom-isoindolin-2-yl)-guanidin
4,7-Dichlor-2-(2-imidazolin-2-yl-methylamino)-isoindolin
4,7-Dichlor-2-(2-imidazolin-2-yl-n-propylamino)-isoindolin

Bevorzugt werden Isoindolinyl-aminoimidazoline der allgemeinen Formel I, in der $R^1$ und $R^2$ die oben angegebene Bedeutung haben, wobei mindestens einer dieser Substituenten $R^1$ und $R^2$ nicht Wasserstoff bedeutet, und $R^3$, $R^4$, $R^7$ und $R^8$ Wasserstoff sind und $R^5$ und $R^6$ zusammen die Äthylengruppe bedeuten, mit der Maßgabe, daß wenn $R^1$ Chlor bedeutet, $R^2$ nicht Wasserstoff ist.

Weiterhin werden Isoindolinyl-guanidine der allgemeinen Formel I bevorzugt, in der $R^1$, $R^2$, $R^3$, $R^4$, $R^7$ und $R^8$ die angegebene Bedeutung haben und $R^5$ und $R^6$ Wasserstoff oder Alkyl mit 1 bis 4 Kohlenstoffatomen bedeuten.

Bevorzugt werden ferner Isoindolinyl-aminoimidazoline der allgemeinen Formel I, in der $R^1$, $R^2$, $R^3$, $R^4$ und $R^7$ die angegebene Bedeutung haben und $R^5$ und $R^6$ zusammen die Äthylengruppe bedeuten und $R^8$ Wasserstoff ist, wobei mindestens einer der Substituenten $R^3$, $R^4$ und $R^7$ Alkyl mit 1 bis 4 Kohlenstoffatomen bedeutet.

Ferner werden Isoindolinyl-aminoimidazoline und Isoindolinyl-guanidine der allgemeinen Formel I bevorzugt, in der $R^8$ eine Alkylgruppe mit 1 bis 4 kohlenstoffatomen, insbesondere die Methylgruppe, darstellt und die übrigen Substituenten die angegebene Bedeutung haben.

Die erfindungsgemäßen neuen Verbindungen und deren Säureadditionssalze besitzen wertvolle therapeutische Eigenschaften. Insbesondere zeichnen sie sich durch eine lang andauernde hypertensive Wirkung an Katzen und Hunden aus. Die Dosierungen liegen bei Katzen im Bereich von 0,1-1 mg/kg. Zur Behandlung hypotoner Zustände beim Menschen werden die Verbindungen in Dosierungen von 1-5 mg pro Tag angewendet.

Weiterhin können die erfindungsgemäßen Substanzen infolge ihrer α-sympathicomimetischen, vasokonstriktorischen Wirkung als Rhinologica, insbesondere in Form von Nasentropfen, verwendet werden. Die Dosierungen betragen bei der bevorzugten Konzentration von 0,1-1 mg/ml, entsprechend

einer 0,01-0,1 %igen wäßrigen Lösung, vorzugsweise mehrere Tropfen pro Nasenloch mehrmals täglich.

Die erfindungsgemäßen Verbindungen besitzen diese hervorragenden Eigenschaften gegenüber anderen, in der Struktur ähnlichen Verbindungen in nicht vorhersehbarer Weise. Man nimmt an, daß diese besonderen, spezifischen Wirkungen von der speziellen Struktur der erfindungsgemäßen Verbindungen abhängen.

Überraschenderweise wurde gefunden, daß insbesondere durch Substitution des Isoindolinrestes in 1,3- bzw. 4,7-Position durch Substituenten der oben angegebenen Bedeutung, die blutdrucksenkende Wirkung der 1,3- bzw. 4,7-unsubstituierten Verbindungen (deutsche Offenlegungsschrift 29 05 501) sich in eine blutdrucksteigernde Wirkung verkehrt. Die überwiegend α-sympathicolytische Wirkung (DE-OS 29 05 501) verändert sich also bei geeigneter Substitution zu einer α-sympathicomimetischen, vasokonstriktorischen Wirkung.

Besonders bevorzugte Verbindungen mit einem besonders hohen therapeutischen Effekt sind :

4,7-Dichlor-2-(2-imidazolin-2-yl-amino)-isoindolin
(4-Chlor-isoindolin-2-yl)-guanidin
(4,7-Dichlor-isoindolin-2-yl)-guanidin

Gemäß der Erfindung werden pharmazeutische Zusammensetzungen geschaffen, die eine Verbindung der Formel I oder deren pharmazeutisch veträgliche Salze, zusammen mit einem pharmazeutisch verträglichen Verdünnungsmittel oder Träger enthalten.

Die Verbindungen gemäß der Erfindung können mit üblichen pharmazeutisch verträglichen Verdünnungsmitteln oder Trägern und gegebenenfalls mit anderen Hilfsmitteln vermischt und beispielsweise oral oder parenteral verabreicht werden. Sie können oral in Form von Tabletten, Dragees, Sirups, Suspensionen und Flüssigkeiten, oder parenteral in Form von Lösungen oder Suspensionen verabreicht werden. Oral zu verabreichende Präparate können ein oder mehrere Zusätze, wie Süßungsmittel, Aromatisierungsmittel, Farbstoffe und Konservierungsmittel, enthalten. Tabletten können den Wirkstoff mit üblichen, pharmazeutisch verträglichen Hilfsmitteln vermischt enthalten, z. B. inerten Verdünnungsmitteln wie Calciumcarbonat, Natriumcarbonat, Lactose und Talk, Granulierungsmitteln und Mitteln, die den Zerfall der Tabletten bei oraler Verabreichung fördern, wie Stärke oder Alginsäure, Bindemitteln wie Stärke oder Gelatine, Gleitmitteln wie Magnesiumstearat, Stearinsäure und Talk.

Geeignete Trägerstoffe sind beispielsweise Milchzucker (Lactose), Gelatine, Maisstärke, Stearinsäure, Äthanol, Propylenglycol, Äther des Tetrahydrofurylalkohols und Wasser.

Die Tabletten können nach bekannten Arbeitsweisen überzogen werden, um den Zerfall und die Resorption im Magen-Darmtrakt zu verzögern, wodurch die Aktivität des Wirkstoffs sich über eine längere Zeitspanne erstrecken kann. Ebenso kann in den Suspensionen der Wirkstoff mit Hilfsmitteln vermischt sein, die für die Herstellung solcher Zusammensetzungen üblich sind, z. B. Suspendiermitteln wie Methylcellulose, Tragacanth oder Natriumalginat, Netzmitteln wie Lecithin, Polyäthylenstearat und Polyoxyäthylensorbitanmonooleat, und Konservierungsmitteln wie Äthylparahydroxybenzoat. Kapseln können den Wirkstoff als einzigen Bestandteil oder vermischt mit einem festen Verdünnungsmittel wie Calciumcarbonat, Calciumphosphat oder Kaolin enthalten. Die injizierbaren Präparate werden ebenfalls in an sich bekannter Weise formuliert. Die pharmazeutischen Präparate können den Wirkstoff in einer Menge von 0,1 bis 90 %, insbesondere 1 bis 90 %, enthalten, wobei der Rest ein Trägerstoff oder Zusatzstoff ist. Im Hinblick auf die Herstellung und Verabreichung werden feste Präparate, wie Tabletten und Kapseln, bevorzugt. Vorzugsweise enthalten die Präparate den Wirkstoff in einer Menge von 1-5 mg zur Behandlung der Hypotonie.

Bei Anwendung als Rhinologica werden die Präparate bevorzugt als Spray oder in Form von Nasentropfen verwendet, bevorzugt als 0,01-0,1 %ige wäßrige Lösungen, denen übliche Verdickungsmittel, Zusatzstoffe und Konservierungsmittel zugegeben sein können.

Die Verbindungen der allgemeinen Formel I sind nach folgenden Verfahren erhältlich :

a) Verbindungen der allgemeinen Formel I mit der oben angegebenen Bedeutung der Reste $R^1$ bis $R^8$ erhält man durch Umsetzung von Verbindungen der allgemeinen Formel II

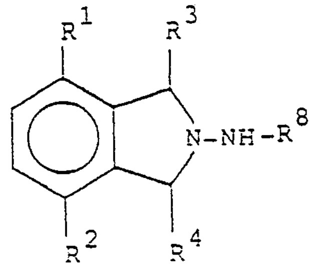

(II)

mit der oben angegebenen Bedeutung von $R^1$, $R^2$, $R^3$, $R^4$ und $R^8$, mit einer Verbindung der allgemeinen

Formel III

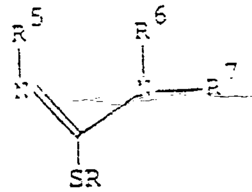

(III)

mit der oben angegebenen Bedeutung von $R^5$, $R^6$ und $R^7$, wobei R Alkyl, vorzugsweise Methyl, bedeutet. Besonders geeignet sind die Methylthioverbindungen in Form des Hydrohalogenids, insbesondere des Hydrojodids. Die Umsetzung wird in einem Alkohol, beispielsweise n-Amylalkohol, als Lösungsmittel bei Siedetemperatur durchgeführt.

b) Isoindolinylimidazoline der allgemeinen Formel I, in der $R^7$ Wasserstoff ist und $R^1$, $R^2$, $R^3$, $R^4$ und $R^8$ die oben angegebene Bedeutung haben, erhält man durch Umsetzung von Verbindungen der allgemeinen Formel II

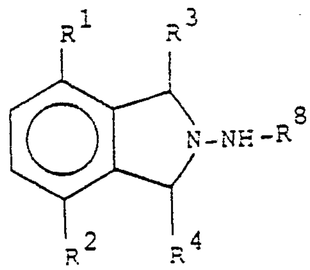

(II)

mit der oben angegebenen Bedeutung der Reste $R^1$ bis $R^4$ und $R^8$, mit einer Verbindung der allgemeinen Formel IV

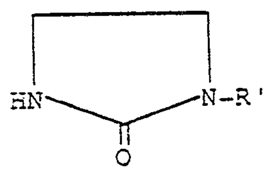

(IV)

in der R' eine Acylgruppe mit 2 bis 4 Kohlenstoffatomen bedeutet, umsetzt und die Acylgruppe abspaltet.

Zweckmäßigerweise führt man die Umsetzung mit einem 1-Acyl-imidazolidin-2-on in Gegenwart von $POCl_3$ bei Temperaturen von 100-120 °C aus. Die Acylgruppe läßt sich mit verdünnten Säuren bei Raumtemperatur abspalten.

c) Isoindolinylguanidine der allgemeinen Formel I, in der die Reste $R^1$ bis $R^8$ die oben angegebene Bedeutung haben, erhält man durch Umsetzung von Verbindungen der allgemeinen Formel II

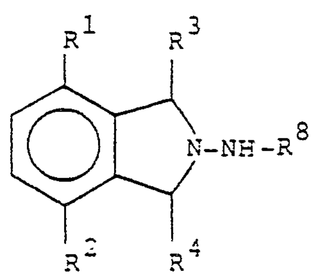

(II)

worin die Reste $R^1$ bis $R^4$ und $R^8$ die oben angegebene Bedeutung haben, mit Cyanamiden. Die Umsetzung erfolgt in Alkoholen, bevorzugt n-Amylalkohol, bei Siedetemperatur. Mit Cyanamid erhält man die mit $R^5 = R^6 = R^7 = H$ unsubstituierten Guanidine, während mit den mit $R^5$, $R^6$ und $R^7$ entsprechend substituierten Cyanamiden die Einführung der Substituenten $R^5$, $R^6$ und $R^7$ gelingt.

d) Isoindolinylguanidine der allgemeinen Formel I, in der die Reste $R^1$ bis $R^8$ die oben angegebene

Bedeutung haben, erhält man durch Reaktion von Verbindungen der allgemeinen Formel II

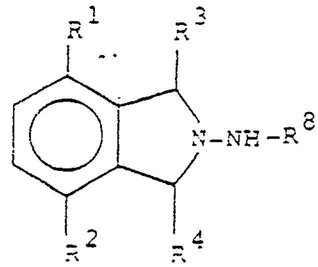

(II)

in der $R^1$, $R^2$, $R^3$, $R^4$ und $R^8$ die oben angegebene Bedeutung haben, mit aliphatischen Isothiocyanaten der Formel $R^5NCS$, mit der oben angegebenen Bedeutung von $R^5$, zu Thiosemicarbaziden der allgemeinen Formel V

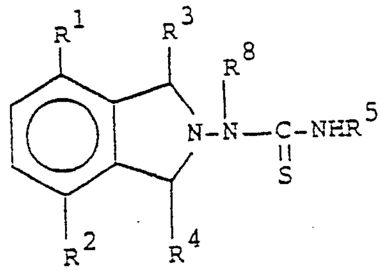

(V)

in der die Reste $R^1$ bis $R^5$ und $R^8$ die oben angegebene Bedeutung haben, anschließende S-Methylierung mit Methyljodid und nachfolgende Umsetzung mit Ammoniak oder mit $R^6$ und $R^7$ substituierten primären oder sekundären Aminen zur Einführung der Reste $R^6$ und $R^7$ unter Abspaltung des Thiosubstituenten.

e) Isoindolinylguanidine der allgemeinen Formel I, in der $R^1$, $R^2$, $R^3$, $R^4$, $R^6$, $R^7$ und $R^8$ die angegebene Bedeutung haben und $R^5$ Wasserstoff ist, erhält man durch Umsetzung von Verbindungen der allgemeinen Formel II

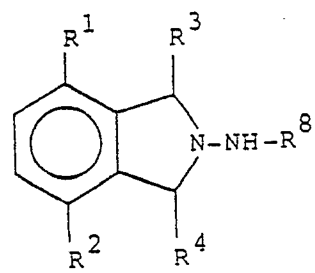

(II)

in der die Reste $R^1$ bis $R^4$ und $R^8$ die angegebene Bedeutung haben, mit Benzoylisothiocyanat zu Thiosemicarbaziden der allgemeinen Formel VI

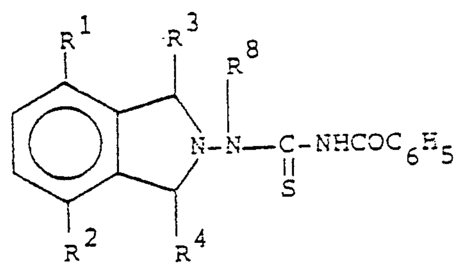

(VI)

anschließende Umwandlung der Thiosemicarbazide zu Verbindungen der allgemeinen Formel VII

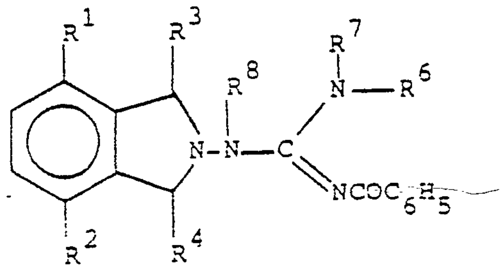

(VII)

in der $R^1$, $R^2$, $R^3$, $R^4$, $R^6$, $R^7$ und $R^8$ die oben angegebene Bedeutung haben, durch Reaktion von Verbindungen (VI) mit Aminen der Formel $R^6R^7NH$ mit der oben angegebenen Bedeutung von $R^6$ und $R^7$ in Gegenwart von Quecksilber (II) oxid in einem geeigneten Lösungsmittel, z. B. Äthanol oder Wasser/Methylenchlorid. Das Quecksilberoxid kann auch durch ein Quecksilbersalz, z. B. $Hg(OAc)_2$, ersetzt werden. Verbindungen der allgemeinen Formel I erhält man aus VII durch Verseifung mit Hilfe einer wässrigen Natrium- oder Kaliumhydroxidlösung bei Siedetemperatur.

f) Isoindolinylguanidine und -imidazoline der allgemeinen Formel I mit der dort angegebenen Bedeutung der Reste $R^1$ bis $R^8$ erhält man durch Verseifung von Verbindungen der allgemeinen Formel VI

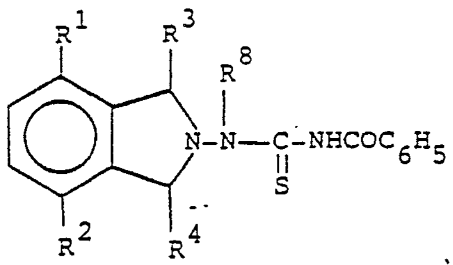

(VI)

mit der angegebenen Bedeutung von $R^1$ bis $R^4$ und $R^8$, mit wäßriger Natrium- oder Kaliumhydroxidlösung zu Thioharnstoffen der allgemeinen Formel VIII

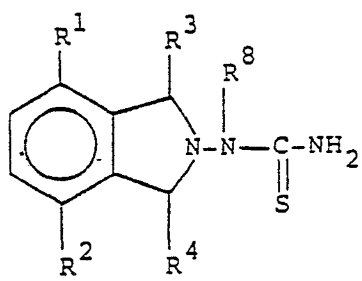

(VIII)

die durch anschließende Alkylierung, vorzugsweise S-Methylierung mit Methyljodid in alkoholischer Lösung, in die S-Alkylisothiuroniumsalze überführt werden, die dann entweder mit $R^6$ und $R^7$ substituierten primären oder sekundären Aminen zu den erfindungsgemäßen Guanidinen oder mit 1,2-Diaminoäthan zu den erfindungsgemäßen Imidazolinen umgesetzt werden.

Die als Ausgangsmaterialien verwendeten Verbindungen der Formel II sind neue Verbindungen. Sie können analog zu bekannten Verbindungen entweder aus entsprechend substituierten Phthalsäureanhydriden oder Xyloldihalogeniden und t-Butylcarbazat hergestellt werden. Sie sind wertvolle Zwischenprodukte bei der Herstellung der erfindungsgemäßen Endprodukte. Die neuen Ausgangsstoffe, die Verfahren zur Herstellung und ihre Verwendung sind ebenfalls Gegenstand der Erfindung.

Die Verbindungen der allgemeinen Formel I können entweder als Basen oder in der Form ihrer Salze aus den Reaktionsgemischen isoliert werden.

Sie lassen sich als Basen mit geeigneten anorganischen oder organischen Säuren nach bekannten Verfahren in Salze überführen. Bevorzugt werden physiologisch verträgliche Salze. Hierfür sind als anorganische Säuren beispielsweise Halogenwasserstoffsäuren, z. B. Salzsäure, oder Schwefelsäure und als organische Säuren z. B. Fumarsäure und Maleinsäure geeignet. Zur Herstellung wird die heiße alkalische Lösung der Base mit der alkoholischen Lösung einer geeigneten Säure versetzt und man erhält nach Ätherzusatz das Salz.

Die folgenden Beispiele zeigen spezielle Ausführungsformen und dienen zur Erläuterung vorliegender Erfindung.

Beispiel 1

4,7-Dichlor-2-(2-imidazolin-2-ylamino)-isoindolin

5,9 g (0,025 M) 2-Amino-4,7-dichlor-isoindolin-hydrochlorid und 3,6 g (0,028 M) 1-Acetyl-imidazolidin-2-on werden in 50 ml Phosphoroxichlorid 2 Stunden auf 100 °C erhitzt. Nach Abdampfen des $POCl_3$ wird der Rückstand in 100 ml Äthanol gelöst und zur Abspaltung der Acetylgruppe eine Stunde zum Sieden erhitzt. Das Lösungsmittel wird anschließend im Vakuum abgezogen, der Rückstand in Wasser aufgenommen und nach Alkalisieren mit verdünnter Natronlauge mit Chloroform extrahiert. Nach Einengen des Chloroforms und Zusatz von Äther erhält man 3,4 g 4,7-Dichlor-2-(2-imidazolin-2-ylamino)-isoindolin als Base, die mit äthanolischer HCl in das Hydrochlorid überführt wird.

Fp. 270-272 °C (Z.).

Herstellungsbeispiel A

4,7-Dimethyl-2-(2-imidazolin-2-ylamino)-isoindolin

Zu einer Suspension von 2,5 g (0,033 M) Ammoniumthiocyanat in 50 ml 1,2-Dimethoxyäthan werden 4,4 g (0,031 M) Benzoylchlorid in 10 ml Dimethoxyäthan getropft, dann wird eine Stunde bei Raumtemperatur gerührt und anschließend werden 4,5 g (0,028 M) 2-Amino-4,7-dimethyl-isoindolin in 30 ml Dimethoxyäthan eingetropft. Nach einstündigem Rühren bei Raumtemperatur wird das ausgefallene Ammonchlorid abgesaugt. Aus dem Filtrat erhält man nach Einengen 5,3 g l-Benzoyl-3-(4,7-dimethyl-isoindolin-2-yl)-thioharnstoff, Fp. 163-164 °C, der durch Verseifen mit 50 ml 10 %iger NaOH in den (4,7-Dimethyl-isoindolin-2-yl)-thioharnstoff überführt wird.

3,0 g des Thioharnstoffs werden in 50 ml Methanol suspendiert und mit 2,8 g Methyljodid eine Stunde zum Sieden erhitzt. Nach üblicher Aufarbeitung erhält man 4,2 g S-Methyl-N-(4,7-dimethyl-isoindolin-2-yl)-isothiuronium-jodid.

Fp. 181-183 °C.

4,0 g des Isothiuroniumsalzes werden in 100 ml n-Amylalkohol suspendiert und mit 1,1 g Äthylendiamin 20 Minuten unter Rückfluß erhitzt. Dann wird das Lösungsmittel abgedampft und der Rückstand zwischen Chloroform und 2 N Natronlauge verteilt. Die organische Phase wird getrocknet und eingedampft. Nach Umkristallisieren aus Ethanol/Essigester erhält man 1,6 g 4,7-Dimethyl-2-(2-imidazolin-2-yl-amino)-isoindolin. Fp. 205-207 °C.

Mit äthanolischer Salzsäure erhält man 1,5 g Hydrochlorid dieser Verbindung.

Fp. 240-242 °C.

Herstellungsbeispiel B

(4,7-Dimethyl-isoindolin-2-yl)-guanidin

3,0 g (0,015 M) 4,7-Dimethyl-isoindolin-hydrochlorid und 0,8 g (0,018 M) Cyanamid werden in 20 ml n-Amylalkohol 2 Stunden zum Sieden erhitzt. Nach Abdampfen des Lösungsmittels wird der Rückstand aus Isopropanol/Äther umkristallisiert. Man erhält 2,7 g des (4,7-dimethyl-isoindolin-2-yl)-guanidins als Hydrochlorid, Fp. 196-198 °C (Z.).

Analog Herstellungsbeispiel B wurden die folgenden erfindungsgemäßen Verbindungen hergestellt (Formel I) :

| Beispiel | $R^1$ | $R^2$ | $R^3$ | $R^4$ | $R^5$ | $R^6$ | $R^7$ | $R^8$ | Fp. °C. | Salz |
|---|---|---|---|---|---|---|---|---|---|---|
| 2 | Cl | Cl | H | H | H | H | H | H | 235-237 | HCl |
| 3 | Cl | H | H | H | H | H | H | H | 244-245 | HCl |

Die neuen Zwischenprodukte der allgemeinen Formel II werden wie folgt hergestellt :

Herstellungsbeispiel C

2-Amino-4,7-dimethyl-isoindolin

a) 52,9 g (0,3 M) 3,6-Dimethylphthalsäureanhydrid in 300 ml DMF werden mit 46,3 g (0,35 M) t-Butylcarbazat 15 Minuten zum Sieden erhitzt. Nach Abdampfen des Lösungsmittels erhält man aus Äthanol 64 g N-(t-Butyloxycarbonylamino)-3,6-dimethyl-phthalimid.

Fp. 187-188 °C (Z.).

b) Zu einer Suspension von 20 g Lithiumaluminiumhydrid in absoluten Tetrahydrofuran werden 63,9 g N-(t-Butyloxycarbonyl-amino)-3,6-dimethyl-phthalimid in 400 ml absoluten Tetrahydrofuran langsam zugetropft. Anschließend wird 2 Stunden zum Sieden erhitzt. Nach üblicher Aufarbeitung erhält man 20 g N-(t-Butyloxycarbonylamino)-4,7-dimethyl-isoindolin.

Fp. 148-151 °C.

c) 20 g N-(t-Butyloxycarbonylamino)-4,7-dimethyl-isoindolin werden in 200 ml konz. Salzsäure eingetragen und 2 Stunden bei Raumtemperatur gerührt. Dabei scheidet sich das Hydrochlorid des 2-Amino-4,7-dimethyl-isoindolins als kristalline Substanz ab. Ausbeute : 17 g.

Fp. 185-187 °C.

Analog Herstellungsbeispiel C wurden folgende 2-Amino-isoindoline (II) hergestellt :

| Beispiel | $R^1$ | $R^2$ | $R^3$ | $R^4$ | $R^8$ | Fp./°C (HCl) |
|---|---|---|---|---|---|---|
| 4 | Cl | H | H | H | H | 205 (Z.) |
| 5 | Cl | Cl | H | H | H | 230-232 (Z.) |

Beispiel 6

Herstellung von Ampullen

Ampullen, die die im folgenden genannten Bestandteile enthalten, lassen sich in bekannter Weise herstellen. Wirkstoffe und Natriumchlorid werden in Wasser gelöst und unter Stickstoff in Glasampullen abgefüllt. Sie können zur Behandlung hypotoner Zustände in einer Dosis von 1 bis 2 Ampullen zweimal täglich verwendet werden.

| | |
|---|---|
| (4,7-Dichlor-isoindolin-2-yl)-guanidin | 1 mg |
| Natriumchlorid | 18 mg |
| dest. Wasser ad | 2,0 ml |

**Patentansprüche** (für die Vertragsstaaten : BE, CH, DE, FR, GB, IT, LI, NL, SE)

1. Isoindolin-2-yl-guanidine und -aminoimidazoline der allgemeinen Formel I

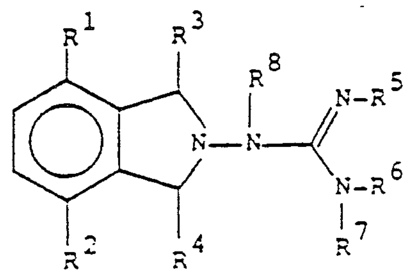

(I)

in der

$R^1$ und $R^2$, die gleich oder verschieden sein können, Wasserstoff oder Halogen bedeuten, wobei mindestens einer der Substituenten $R^1$, $R^2$ Halogen ist,

$R^3$ und $R^4$, die gleich oder verschieden sein können Wasserstoff oder Alkyl mit 1 bis 4 Kohlenstoffatomen,

$R^5$, $R^6$, $R^7$ und $R^8$, die gleich oder verschieden sein können, Wasserstoff oder Alkyl mit 1 bis 4 Kohlenstoffatomen oder

$R^5$ und $R^6$ zusammen die Äthylengruppe bedeuten,

sowie deren Salze, ausgenommen 2-(N-Amino-isoindolinyl)-imidazolin und 4-Chlor-2-(2-imidazolin-2-ylamino)-isoindolin.

2. Isoindolinyl-aminoimidazoline nach Anspruch 1, gemäß der allgemeinen Formel I, in der $R^1$ und $R^2$ die oben angegebene Bedeutung haben, wobei mindestens einer dieser Substituenten $R^1$ und $R^2$ nicht Wasserstoff bedeutet, und $R^3$ und $R^4$, $R^7$ und $R^8$ Wasserstoff sind und $R^5$ und $R^6$ zusammen die Äthylengruppe bedeuten, mit der Maßgabe, daß wenn $R^1$ Chlor bedeutet, $R^2$ nicht Wasserstoff ist.

3. Isoindolinyl-guanidine nach Anspruch 1, gemäß der allgemeinen Formel I, in der $R^1$, $R^2$, $R^3$, $R^4$, $R^7$

und $R^8$ die angegebene Bedeutung haben und $R^5$ und $R^6$ Wasserstoff oder Alkyl mit 1 bis 4 Kohlenstoffatomen bedeuten.

4. Isoindolinyl-aminoimidazoline nach Anspruch 1, gemäß der allgemeinen Formel I, in der $R^1$, $R^2$, $R^3$, $R^4$ und $R^7$ die angegebene Bedeutung haben und $R^5$ und $R^6$ zusammen die Äthylengruppe bedeuten und $R^8$ Wasserstoff ist, wobei mindestens einer der Substituenten $R^3$, $R^4$ und $R^7$ Alkyl mit 1 bis 4 Kohlenstoffatomen bedeutet.

5. Isoindolinyl-aminoimidazoline und Isoindolinyl-guanidine nach Anspruch 1, gemäß der allgemeinen Formel I, in der $R^8$ eine Alkylgruppe mit 1 bis 4 Kohlenstoffatomen, darstellt und die übrigen Substituenten die angegebene Bedeutung haben.

6. Verfahren zur Herstellung der Isoindolinylguanidinen und -imidazolinen gemäß der allgemeinen Formel I mit der oben angegebenen Bedeutung der Rest $R^1$ bis $R^8$, dadurch gekennzeichnet, daß man Verbindungen der allgemeinen Formel II

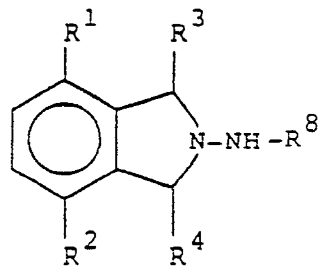

(II)

mit der oben gegebenen Bedeutung von $R^1$, $R^2$, $R^3$, $R^4$ und $R^8$, mit einer Verbindung der allgemeinen Formel III

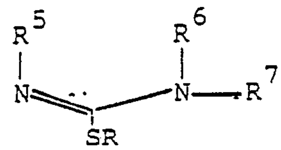

(III)

mit der oben angegebenen Bedeutung von $R^5$, $R^6$ und $R^7$, worin R eine Alkylgruppe bedeutet, umsetzt.

7. Verfahren zur Herstellung von Isoindolinylimidazolinen gemäß der allgemeinen Formel I, in der $R^7$ Wasserstoff ist und $R^1$, $R^2$, $R^3$, $R^4$ und $R^8$ die angegebene Bedeutung haben, dadurch gekennzeichnet, daß man Verbindungen der allgemeinen Formel II

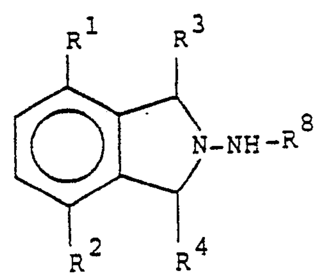

(II)

mit der angegebenen Bedeutung der Reste $R^1$ bis $R^4$ und $R^8$, mit einer Verbindung der allgemeinen Formel IV

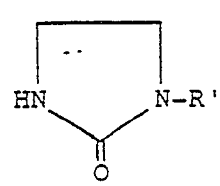

(IV)

in der R' eine Acylgruppe mit 2 bis 4 Kohlenstoffatomen bedeutet, umsetzt und die Acylgruppe abspaltet.

8. Verfahren zur Herstellung von Isoindolinylguanidinen gemäß der allgemeinen Formel I, in der die Reste $R^1$ bis $R^8$ die angegebene Bedeutung haben, dadurch gekennzeichnet, daß man Verbindungen der allgemeinen Formel II

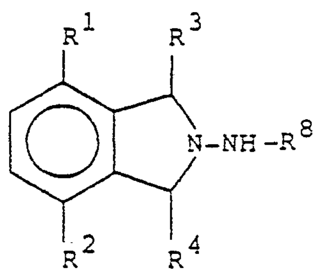

(II)

mit der oben angegebenen Bedeutung von $R^1$ bis $R^4$ und $R^8$, mit Cyanamid oder mit $R^5$, $R^6$ und $R^7$ entsprechend substituierten Cyanamiden umsetzt.

9. Verfahren zur Herstellung von Isoindolinylguanidinen gemäß der allgemeinen Formel I, in der die Reste $R^1$ bis $R^8$ die angegebene Bedeutung haben, dadurch gekennzeichnet, daß man Verbindungen der allgemeinen Formel II

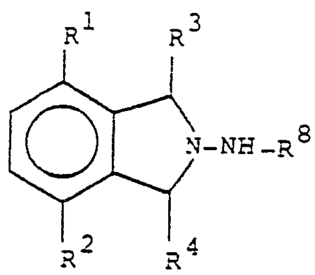

(II)

in der $R^1$, $R^2$, $R^3$, $R^4$ und $R^8$ die oben angegebene Bedeutung haben, mit aliphatischen Isothiocyanaten der Formel $R^5NCS$, mit der oben angegebenen Bedeutung von $R^5$, zu Thiosemicarbaziden der allgemeinen Formel V

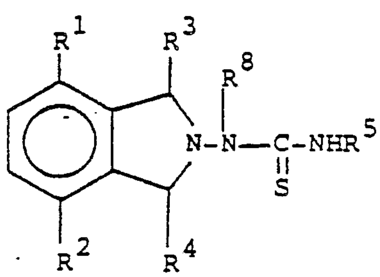

(V)

umsetzt, in der $R^1$, $R^2$, $R^3$, $R^4$, $R^5$ und $R^8$ die oben angegebene Bedeutung haben, diese Thiosemicarbazide einer S-Methylierung mit Methyljodid unterwirft und die erhaltenen Reaktionsprodukte anschließend mit Ammoniak, primären oder sekundären Aminen umsetzt.

10. Verfahren zur Herstellung von Isoindolinylguanidinen gemäß der allgemeinen Formel I, in der die Reste $R^1$, $R^2$, $R^3$, $R^4$, $R^6$, $R^7$ und $R^8$ die angegebene Bedeutung haben und $R^5$ Wasserstoff ist, dadurch gekennzeichnet, daß man Verbindungen der allgemeinen Formel II

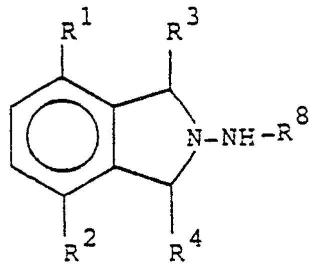

(II)

mit der oben angegebenen Bedeutung der Reste $R^1$ bis $R^4$ und $R^8$ mit Benzoylisothiocyanat zu Thiosemicarbaziden der allgemeinen Formel VI umsetzt.

(Siehe Formel VI Seite 11 f.)

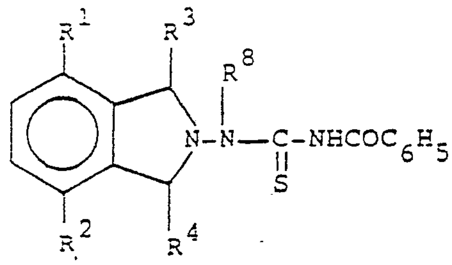

(VI)

die durch Reaktion mit Aminen der allgemeinen Formel $R^6R^7NH$, mit der oben angegebenen Bedeutung von $R^6$ und $R^7$, in die Verbindungen der allgemeinen Formel VII

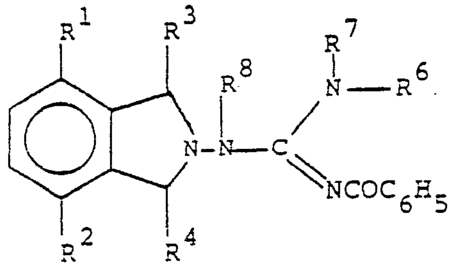

(VII)

überführt werden, und anschließend die Benzoylgruppe abspaltet.

11. Verfahren zur Herstellung von Isoindolinylguanidinen und -imidazolinen gemäß der allgemeinen Formel I mit der dort angegebenen Bedeutung der Reste $R^1$ bis $R^8$, dadurch gekennzeichnet, daß man Verbindungen der allgemeinen Formel VI

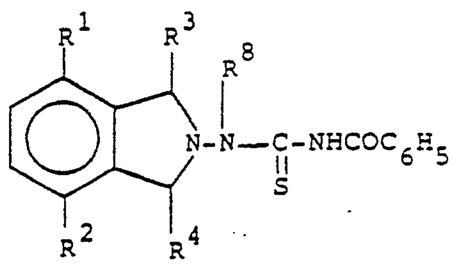

(VI)

mit der oben angegebenen Bedeutung der Reste $R^1$ bis $R^4$ und $R^8$, durch Abspalten des Benzoylrests zu den entsprechenden Thioharnstoffen der Formel VIII umsetzt,

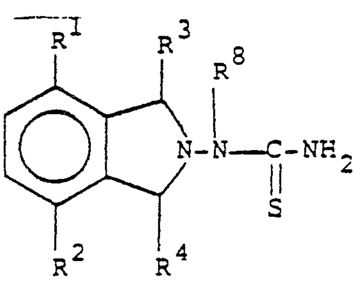

(VIII)

die durch Alkylierung in die S-Alkylisothiuroniumsalze überführt werden, und diese Verbindungen entweder mit primären oder sekundären Aminen zu Guanidinen oder mit 1,2-Diaminoäthan zu den Imidazolinen umsetzt.

12. Pharmazeutisches Präparat, dadurch gekennzeichnet, daß es eine oder mehrere der Verbindungen gemäß Anspruch 1 oder deren physiologisch verträgliche Salze und gegebenenfalls übliche Trägerstoffe und/oder Verdünnungsmittel enthält.

13. Verbindungen der allgemeinen Formel I oder deren physiologisch verträgliche Säureadditionssalze gemäß Anspruch 1 zur Anwendung bei hypotonen Zuständen.

14. Verbindungen der allgemeinen Formel I oder deren physiologisch verträgliche Säureadditionssalze zur Anwendung als Rhinologica.

15. (4,7-Dichlor-isoindolin-2-yl)-guanidine

16. 4,7-Dichlor-2-(2-imidazolin-2-ylamino)-isoindolin

17. Isoindolinyl-aminoimidazoline und Isoindolinyl-guanidine nach Anspruch 1, gemäß der allgemeinen Formel I, in der $R^8$ die Methylgruppe darstellt und die übrigen Substituenten die angegebene

Bedeutung haben.

**Patentansprüche** (für den Vertragsstaat AT)

1. Verfahren zur Herstellung von Isoindolin-2-yl-guanidinen und -aminoimidazolinen der allgemeinen Formel I

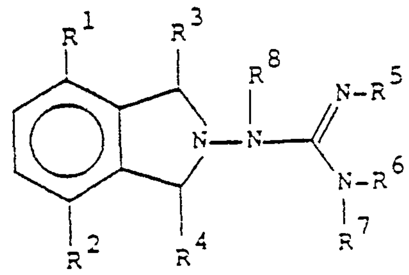

(I)

in der

$R^1$ und $R^2$, die gleich oder verschieden sein können, Wasserstoff oder Halogen bedeuten, wobei mindestens einer der Substituenten $R^1$, $R^2$ Halogen ist,

$R^3$ und $R^4$, die gleich oder verschieden sein können, Wasserstoff, oder Alkyl mit 1 bis 4 Kohlenstoffatomen,

$R^5$, $R^6$, $R^7$ und $R^8$, die gleich oder verschieden sein können, Wasserstoff oder Alkyl mit 1 bis 4 Kohlenstoffatomen oder

$R^5$ und $R^6$ zusammen die Äthylengruppen bedeuten,

sowie deren Salzen, ausgenommen 4-Chlor-2-(2-imidazolin-2-ylamino)-isoindolin, dadurch gekennzeichnet, daß man eine Verbindung der Formel II

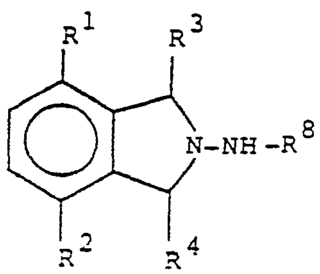

(II)

mit der oben angegebenen Bedeutung von $R^1$, $R^2$, $R^3$, $R^4$ und $R^8$, mit einer Verbindung der allgemeinen Formel III

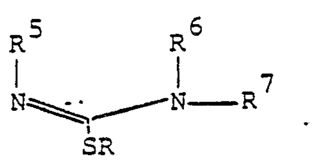

(III)

mit der oben angegebenen Bedeutung von $R^5$, $R^6$ und $R^7$, worin R eine Alkylgruppe bedeutet, umsetzt und gewünschtenfalls die freien Basen mit Säuren in die entsprechenden Säureadditionssalze umwandelt und

a) zur Herstellung von Isoindolinylimidazolinen gemäß der allgemeinen Formel I, in der $R^7$ Wasserstoff ist und $R^1$, $R^2$, $R^3$, $R^4$ und $R^8$ die angegebene Bedeutung haben, Verbindungen der allgemeine Formel II

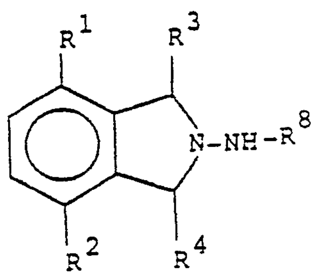

(II)

mit der angegebenen Bedeutung der Reste $R^1$ bis $R^4$ und $R^8$, mit einer Verbindung der allgemeinen Formel IV

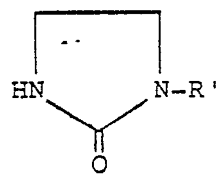

(IV)

in der R' eine Acylgruppe mit 2 bis 4 Kohlenstoffatomen bedeutet, umsetzt und die Acylgruppe abspaltet und gewünschtenfalls die freien Basen mit Säuren in die entsprechenden Säureadditionssalze umwandelt und

b) zur Herstellung von Isoindolinylguanidinen gemäß der allgemeinen Formel I, in der die Reste $R^1$ bis $R^8$ die angegebene Bedeutung haben, Verbindungen der allgemeinen Formel II

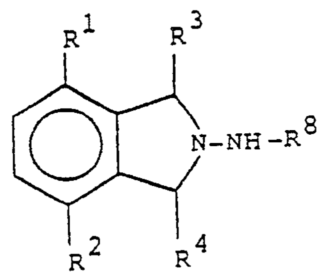

(II)

mit der oben angegebenen Bedeutung von $R^1$ bis $R^4$ und $R^8$, mit Cyanamid oder mit $R^5$, $R^6$ und $R^7$ entsprechend substituierten Cyanamiden umsetzt und gewünschtenfalls die freien Basen mit Säuren in die entsprechenden Säureadditionssalze umwandelt und

c) zur Herstellung von Isoindolinylguanidinen gemäß der allgemeinen Formel I, in der die Reste $R^1$ bis $R^8$ die angegebene Bedeutung haben, Verbindungen der allgemeinen Formel II

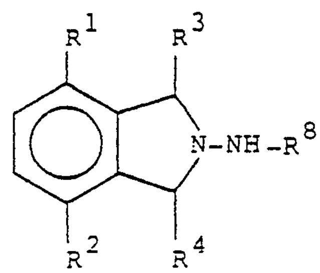

(II)

in der $R^1$, $R^2$, $R^3$, $R^4$ und $R^8$ die oben angegebene Bedeutung haben, mit aliphatischen Isothiocyanaten der Formel $R^5NCS$, mit der oben angegebenen Bedeutung von $R^5$, zu Thiosemicarbaziden der allgemeinen Formel V

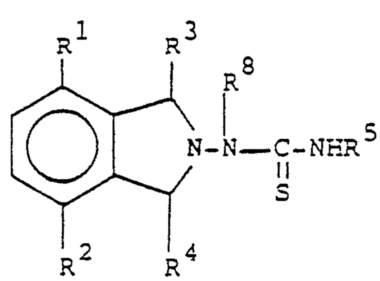

(V)

umsetzt, in der $R^1$, $R^2$, $R^3$, $R^4$, $R^5$ und $R^8$ die oben angegebene Bedeutung haben, diese Thiosemicarbazide einer S-Methylierung mit Methyljodid unterwirft und die erhaltenen Reaktionsprodukte anschließend mit Ammoniak oder mit $R^6$ und $R^7$ substituierten primären oder sekundären Aminen umsetzt und gewünschtenfalls die freien Basen mit Säuren in die entsprechenden Säureadditionssalze umwandelt und

d) zur Herstellung von Isoindolinylguanidinen gemäß der allgemeinen Formel I, in der die Reste $R^1$, $R^2$, $R^3$, $R^4$, $R^6$, $R^7$ und $R^8$ die angegebene Bedeutung haben und $R^5$ Wasserstoff ist, Verbindungen der allgemeinen Formel II

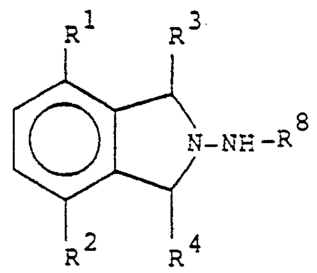

(II)

mit der oben angegebenen Bedeutung der Reste $R^1$ bis $R^4$ und $R^8$ mit Benzoylisothiocyanat zu Thiosemicarbaziden der allgemeinen Formel VI umsetzt,

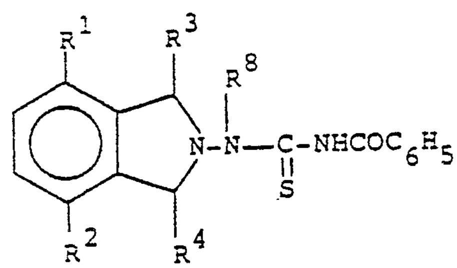

(VI)

die durch Reaktion mit Aminen der allgemeinen Formel $R^6R^7NH$, mit der oben angegebenen Bedeutung von $R^6$ und $R^7$, in die Verbindungen der allgemeinen Formel VII

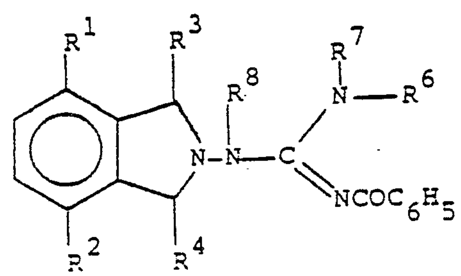

(VII)

überführt werden, und anschließend die Benzoylgruppe abspaltet und gewünschtenfalls die freien Basen mit Säuren in die entsprechenden Säureadditionssalze umwandelt und

e) zur Herstellung von Isoindolinylguanidinen und -imidazolinen gemäß der allgemeinen Formel I mit der dort angegebenen Bedeutung der Reste $R^1$ bis $R^8$, Verbindungen der allgemeinen Formel VI

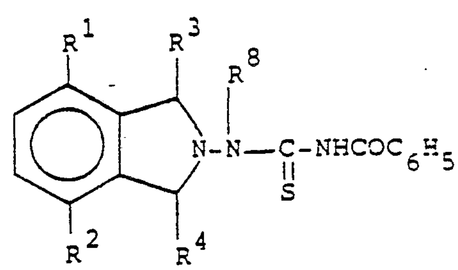

(VI)

mit der oben angegebenen Bedeutung der Reste $R^1$ bis $R^4$ und $R^8$, durch Abspalten des Benzoylrests zu

den entsprechenden Thioharnstoffen der Formel VIII umsetzt,

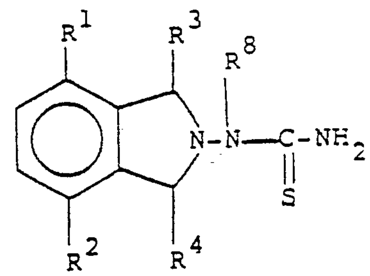

(VIII)

die durch Alkylierung in die S-Alkylisothiuroniumsalze überführt werden und diese Verbindungen entweder mit $R^6$ und $R^7$ substituierten primären oder sekundären Aminen zu Guanidinen oder mit 1,2-Diaminoäthan zu den Imidazolinen umsetzt und gewünschtenfalls die freien Basen mit Säuren in die entsprechenden Säureadditionssalze umwandelt.

2. Verfahren nach Anspruch 1 zur Herstellung von (4,7-Dichlor-isoindolin-2-yl)-guanidin, dadurch gekennzeichnet, daß man 4,7-Dichlor-isoindolin mit Cyanamid umsetzt.

3. Verfahren zur Herstellung von Arzneimitteln, dadurch gekennzeichnet, daß man eine oder mehrere Verbindungen der allgemeinen Formel I oder deren physiologisch verträglichen Säureadditionssalze mit üblichen galenischen Hilfs-, Träger- oder Schmiermitteln bzw. Substanzen zur Erzielung einer Depotwirkung zu Tabletten, Kapseln oder Lösungen formuliert.

**Claims** (for the Contracting States : BE, CH, DE, FR, GB, IT, LI, NL, SE)

1. Isoindolin-2-ylguanidines and isoindolin-2-ylaminoimidazolines of the general formula I

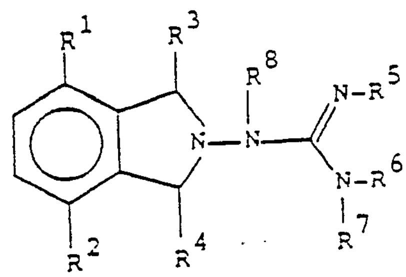

(I)

in which

$R^1$ and $R^2$, which can be identical or different, denote hydrogen or halogen, at least one of the substituents $R^1$ and $R^2$ being halogen,

$R^3$ and $R^4$, which can be identical or different, denote hydrogen or alkyl having 1 to 4 carbon atoms, and

$R^5$, $R^6$, $R^7$ and $R^8$, which can be identical or different, denote hydrogen or alkyl having 1 to 4 carbon atoms, or

$R^5$ and $R^6$ together denote the ethylene group,

and salts thereof, excepting 2-(N-aminoisoindolinyl)-imidazoline and 4-chloro-2-(2-imidazolin-2-ylamino)-isoindoline.

2. Isoindolinylaminoimidazolines according to Claim 1, of the general formula I in which $R^1$ and $R^2$ have the meaning indicated above, at least one of these substituents $R^1$ and $R^2$ not denoting hydrogen, and $R^3$ and $R^4$, and $R^7$ and $R^8$ are hydrogen and $R^5$ and $R^6$ together denote the ethylene group, subject to the proviso that $R^2$ is not hydrogen if $R^1$ denotes chlorine.

3. Isoindolinylguanidines according to Claim 1, of the general formula I in which $R^1$, $R^2$, $R^3$, $R^4$, $R_7$ and $R^8$ have the meaning indicated and $R^5$ and $R^6$ denote hydrogen or alkyl having 1 to 4 carbon atoms.

4. Isoindolinylaminoimidazolines according to Claim 1, of the general formula I in which $R^1$, $R^2$, $R^3$, $R^4$ and $R^7$ have the meaning indicated and $R^5$ and $R^6$ together denote the ethylene group and $R^8$ is hydrogen, at least one of the substituents $R^3$, $R^4$ and $R^7$ denoting alkyl having 1 to 4 carbon atoms.

5. Isoindolinylaminoimidazolines and isoindolinylguanidines according to Claim 1, of the general formula I in which $R^8$ represents an alkyl group having 1 to 4 carbon atoms, and the remaining substituents have the meaning indicated.

6. Process for the preparation of the isoindolinylguanidines and isoindolinylimidazolines of the general formula I in which the radicals $R^1$ to $R^8$ have the meaning indicated above, characterized in that compounds of the general formula II

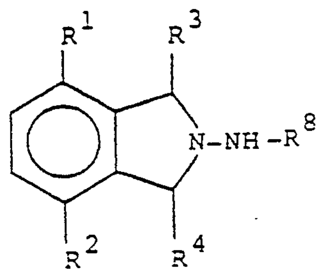 (II)

in which $R^1$, $R^2$, $R^3$, $R^4$ and $R^8$ have the meaning given above, are reacted with a compound of the general formula III

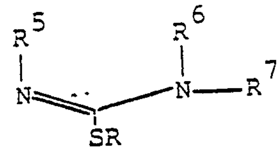 (III)

in which $R^5$, $R^6$ and $R^7$ have the meaning indicated above, R denoting an alkyl group.

7. Process for the preparation of isoindolinylimidazolines of the general formula I in which $R^7$ is hydrogen and $R^1$, $R^2$, $R^3$, $R^4$ and $R^8$ have the meaning indicated, characterized in that compounds of the general formula II

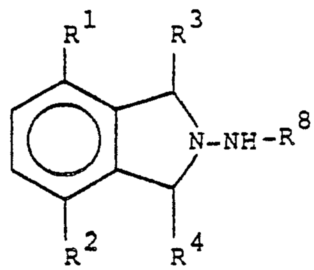 (II)

in which the radicals $R^1$ to $R^4$ and $R^8$ have the meaning indicated, are reacted with a compound of the general formula IV

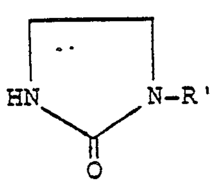 (IV)

in which R' denotes an acyl group having 2 to 4 carbon atoms, and the acyl group is split off.

8. Process for the preparation of isoindolinylguanidines of the general formula I in which the radicals $R^1$ to $R^8$ have the meaning indicated, characterized in that compounds of the general formula II

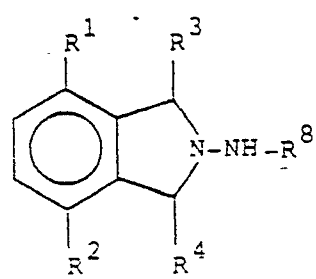 (II)

in which $R^1$ to $R^4$ and $R^8$ have the meaning indicated above, are reacted with cyanamide or with cyanamides which are appropriately substituted by $R^5$, $R^6$ and $R^7$.

9. Process for the preparation of isoindolinylguanidines of the general formula I in which the radicals

$R^1$ to $R^8$ have the meaning indicated, characterized in that compounds of the general formula II

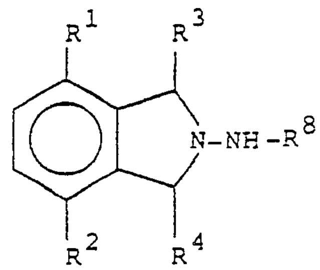

(II)

in which $R^1$, $R^2$, $R^3$, $R^4$ and $R^8$ have the meaning indicated, are reacted with aliphatic isothiocyanates of the formula $R^5NCS$ in which $R^5$ has the meaning indicated above, to give thiosemicarbazides of the general formula V

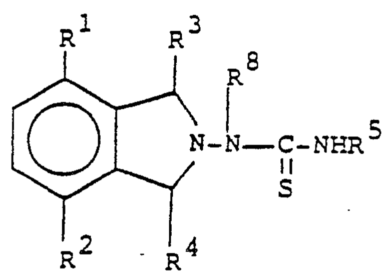

(V)

in which $R^1$, $R^2$, $R^3$, $R^4$, $R^5$ and $R^8$ have the meaning indicated above, these thiosemicarbazides are subjected to an S-methylation with methyl iodide, and the reaction products obtained are then reacted with ammonia or primary or secondary amines.

10. Process for the preparation of isoindolinylguanidines of the general formula I in which the radicals $R^1$, $R^2$, $R^3$, $R^4$, $R^6$, $R^7$ and $R^8$ have the meaning indicated and $R^5$ is hydrogen, characterized in that compounds of the general formula II

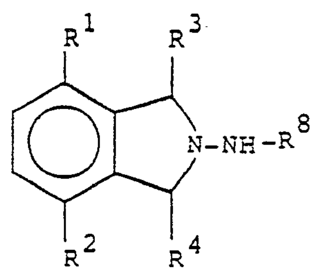

(II)

in which the radicals $R^1$ to $R^4$ and $R^8$ have the meaning indicated above, are reacted with benzoyl isothiocyanate to give thiosemicarbazides of the general formula VI

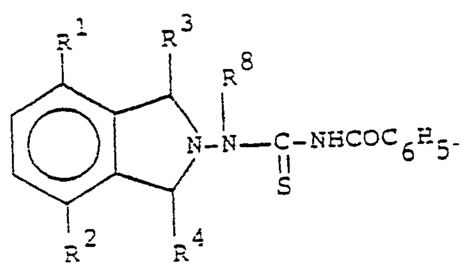

(VI)

which are converted, by reaction with amines of the general formula $R^6R^7NH$ in which $R^6$ and $R^7$ have the meaning indicated above, into the compounds of the general formula VII

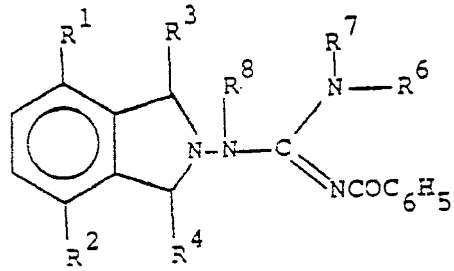

(VII)

and the benzoyl group is then split off.

11. Process for the preparation of isoindolinylguanidines and isoindolinylimidazolines of the general formula I in which the radicals $R^1$ to $R^8$ have the meaning indicated in that formula, characterized in that compounds of the general formula VI

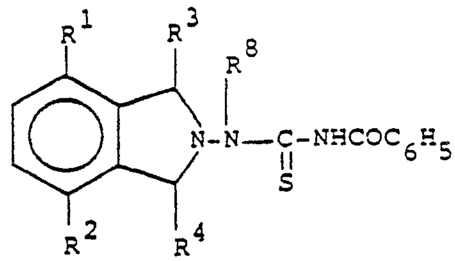

(VI)

in which the radicals $R^1$ to $R^4$ and $R^8$ have the meaning indicated above, are converted, by splitting off the benzoyl radical, into the corresponding thioureas of the formula VIII

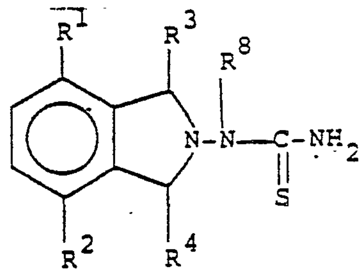

(VIII)

which are converted by alkylation into the S-alkylisothiuronium salts, and these compounds are reacted, either with primary or secondary amines to give guanidines, or with 1,2-diaminoethane to give the imidazolines.

12. Pharmaceutical formulation characterized in that it contains one or more of the compounds according to Claim 1 or physiologically acceptable salts thereof and, if appropriate, customary excipients and/or diluents.

13. Compounds of the general formula I or physiologically acceptable acid addition salts thereof according to Claim 1 for use in hypotensive states.

14. Compounds of the general formula I or physiologically acceptable acid addition salts thereof for use as rhinological agents.

15. (4,7-Dichloroisoindolin-2-yl)-guanidines.

16. 4,7-Dichloro-2-(2-imidazolin-2-ylamino)-isoindoline.

17. Isoindolinylaminoimidazolines and isoindolinylguanidines according to Claim 1, of the general formula I in which $R^8$ represents the methyl group and the other substituents have the meaning indicated.

**Claims** (for the Contracting State AT)

1. Process for the preparation of isoindolin-2-yl-guanidines and isoindolin-2-ylaminoimidazolines of the general formula I

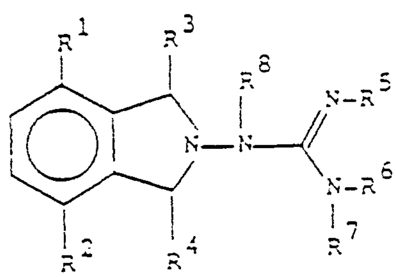

(I)

in which

$R^1$ and $R^2$, which can be identical or different, denote hydrogen or halogen, at least one of the substituents $R^1$ and $R^2$ being halogen,

$R^3$ and $R^4$, which can be identical or different, denote hydrogen or alkyl having 1 to 4 carbon atoms, and

$R^5$, $R^6$, $R^7$ and $R^8$, which can be identical or different, denote hydrogen or alkyl having 1 to 4 carbon atoms, or

$R^5$ and $R^6$ together denote the ethylene group,

and salts thereof, excepting 4-chloro-2-(2-imidazolin-2-ylamino)-isoindoline, characterized in that a compound of the formula II

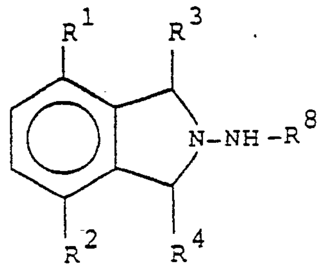

(II)

in which $R^1$, $R^2$, $R^3$, $R^4$ and $R^8$ have the meaning indicated above, is reacted with a compound of the general formula III

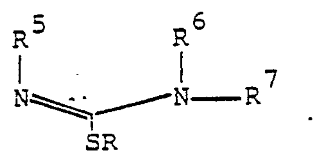

(III)

in which $R^5$, $R^6$ and $R^7$ have the meaning indicated, R denoting an alkyl group, and, if desired, the free bases are converted into the corresponding acid addition salts by means of acids, and

a) in order to prepare isoindolinylimidazolines according to the general formula I in which $R^7$ is hydrogen and $R^1$, $R^2$, $R^3$, $R^4$ and $R^8$ have the meaning indicated, compounds of the general formula II

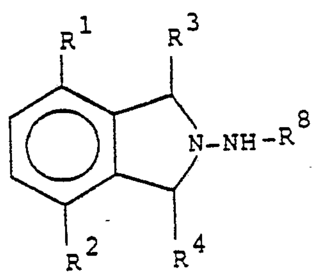

(II)

in which the radicals $R^1$ to $R^4$ and $R^8$ have the meaning indicated, are reacted with a compound of the general formula IV

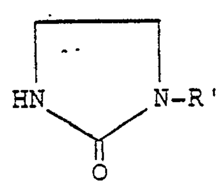

(IV)

in which R' denotes an acyl group having 2 to 4 carbon atoms, and the acyl group is split off and, if desired, the free bases are converted into the corresponding acid addition salts by means of acids, and

b) in order to prepare isoindolinylguanidines according to the general formula I in which the radicals $R^1$ to $R^8$ have the meaning indicated, compounds of the general formula II

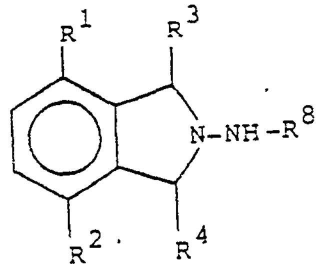

(II)

in which $R^1$ to $R^4$ and $R^8$ have the meaning indicated above, are reacted with cyanamide or with cyanamides which are appropriately substituted by $R^5$, $R^6$ and $R^7$ and, if desired, the free bases are converted into the corresponding acid addition salts by means of acids, and

c) in order to prepare isoindolinylguanidines according to the general formula I in which the radicals $R^1$ to $R^8$ have the meaning indicated, compounds of the general formula II

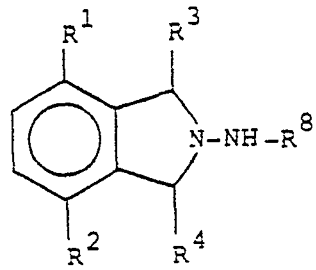

(II)

in which $R^1$, $R^2$, $R^3$, $R^4$ and $R^8$ have the meaning indicated above, are reacted with aliphatic isothiocyanates of the formula $R^5NCS$ in which $R^5$ has the meaning indicated above, to give thiosemicarbazides of the general formula V

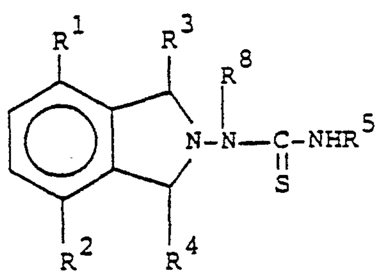

. (V)

in which $R^1$, $R^2$, $R^3$, $R^4$, $R^5$ and $R^8$ have the meaning indicated above, these thiosemicarbazides are subjected to an S-methylation with methyl iodide, and the reaction products obtained are then reacted with ammonia or primary or secondary amines which are substituted by $R^6$ and $R^7$ and, if desired, the free bases are converted into the corresponding acid addition saltz by means of acids, and

d) in order to prepare isoindolinylguanidines according to the general formula I in which the radicals $R^1$, $R^2$, $R^3$, $R^4$, $R^6$, $R^7$ and $R^8$ have the meaning indicated and $R^5$ is hydrogen, compounds of the general formula II

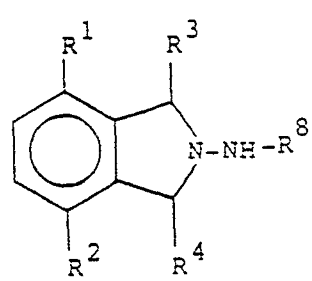

(II)

in which the radicals $R^1$ to $R^4$ and $R^8$ have the meaning indicated above, are reacted with benzoyl isothiocyanate to give thiosemicarbazides of the general formula VI

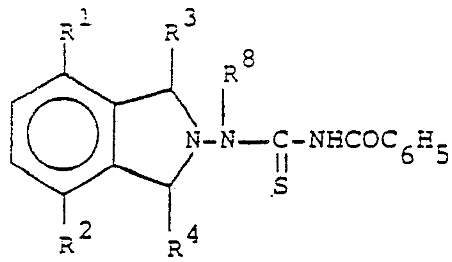

(VI)

which are converted, by reaction with amines of the general formula $R^6R^7NH$ in which $R^6$ and $R^7$ have the meaning indicated above, into the compounds of the general formula VII

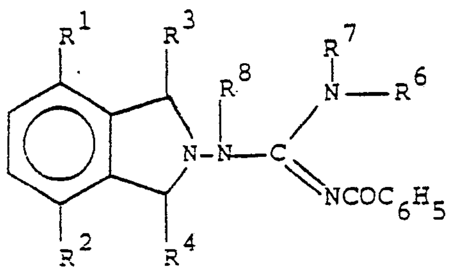

(VII)

and the benzoyl group is then split off and, if desired, the free bases are converted into the corresponding acid addition salts by means of acids, and

e) in order to prepare isoindolinylguanidines and isoindolinylimidazolines according to the general formula I in which the radicals $R^1$ to $R^8$ have the meaning indicated in that formula, compounds of the general formula VI

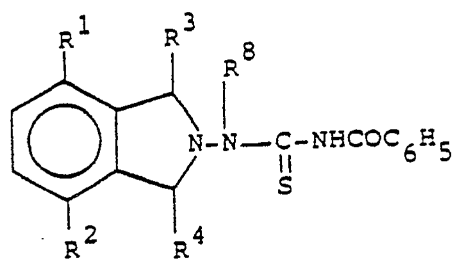

(VI)

in which the radicals $R^1$ to $R^4$ and $R^8$ have the meaning indicated above, are converted, by splitting off the benzoyl radical, into the corresponding thioureas of the formula VIII

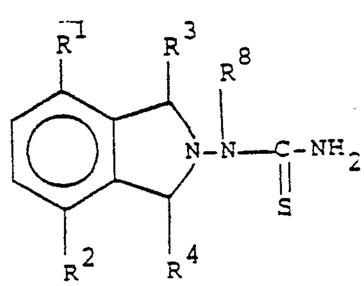

(VIII)

which are converted by alkylation into the S-alkylisothiuronium salts, and these compounds are reacted, either with primary or secondary amines substituted by $R^6$ and $R^7$ to give guanidines, or with 1,2-diaminoethane to give the imidazolines, and, if desired, the free bases are converted into the corresponding acid addition salts by means of acids.

2. Process according to Claim 1 for the preparation of (4,7-dichloroisoindolin-2-yl)-guanidine,

characterized in that 4,7-dichloroisoindoline is reacted with cyanamide.

3. Process for the preparation of medicaments, characterized in that one or more compounds of the general formula I or physiologically acceptable acid addition salts thereof are formulated, with customary pharmaceutical auxiliaries, excipients or lubricants or substances for achieving a depot action, to give tablets, capsules or solutions.

**Revendications** (pour les Etats contractants : BE, CH, DE, FR, GB, IT, LI, NL, SE)

1. Isoindolin-2-yl-guanadines et -aminoimidazolines de formule générale I

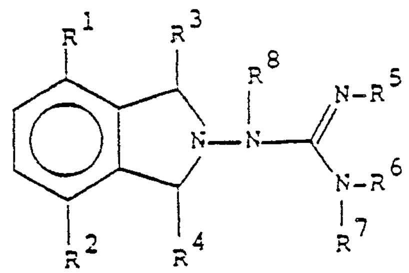

(I)

dans laquelle

$R^1$ et $R^2$ qui peuvent être identiques ou différents, représentent de l'hydrogène ou un halogène, dans lesquelles au moins l'un des substituants $R^1$, $R^2$ est un halogène,

$R^3$ et $R^4$, qui peuvent être identiques ou différents, représentent l'hydrogène ou un alkyle de 1 à 4 atomes de carbone.

$R^5$, $R^6$, $R^7$ et $R^8$ qui peuvent être identiques ou différents, représentent de l'hydrogène ou un alkyle de 1 à 4 atomes de carbone ou

$R^5$ et $R^6$ représentent ensemble le groupe éthylène,

ainsi que leurs sels à l'exception de la (N-Aminoisoindolinyl)-2-imidazoline et de la chloro-4-(imidazolin-2-yl-amino-2)-2-isoindoline.

2. Isoindolinyl-aminoimidazolines selon la revendication 1, conformément à la formule générale I, dans laquelle $R^1$ et $R^2$ ont la signification donnée plus haut, dans laquelle au moins l'un de ces substituants $R^1$ et $R^2$ n'est pas l'hydrogène et $R^3$ et $R^4$, $R^7$ et $R^8$ sont l'hydrogène et $R^5$ et $R^6$ forment ensemble le groupe éthylène, avec la condition que lorsque $R^1$ représente le chlore, $R^2$ n'est pas l'hydrogène.

3. Isoindolinyl-guanidines selon la revendication 1, conformément à la formule générale I, dans laquelle $R^1$, $R^2$, $R^3$, $R^4$, $R^7$ et $R^8$ ont la signification donnée et $R^5$ et $R^6$ représentent l'hydrogène ou un alkyle de 1 à 4 atomes de carbone.

4. Isoindolinyl-aminoimidazolines selon la revendication 1, conformément à la formule générale I, dans laquelle $R^1$, $R^2$, $R^3$, $R^4$ et $R^7$ ont la signification donnée, et $R^5$ et $R^6$ sont ensemble le groupe éthylène et $R^8$ est l'hydrogène, dans laquelle au moins l'un des substituants $R^3$, $R^4$ et $R^7$ représente un alkyle de 1 à 4 atomes de carbone.

5. Isoindolinyl-aminoimidazolines et isoindolinylguanidines selon la revendication 1, conformément à la formule générale I, dans laquelle $R^8$ représente un groupe alkyle de 1 à 4 atomes de carbone, et les substituants restants ont la signification donnée.

6. Procédé de préparation de l'isoindolinylguanidines et -imidazolines conformément à la formule générale I avec la signification donnée ci-dessus pour les restes $R^1$ à $R^8$, caractérisé en ce que l'on fait réagir des composés de formule générale II

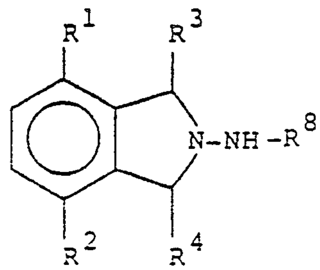

(II)

avec la signification de $R^1$, $R^2$, $R^3$, $R^4$ et $R^8$ donnée ci-dessus, avec un composé de formule générale III

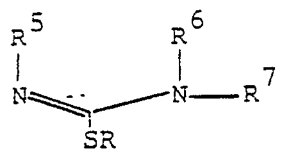

(III)

avec la signification de $R^5$, $R^6$ et $R^7$ donnée ci-dessus dans laquelle R représente un groupe alkyle.

7. Procédé de préparation d'isoindolinylimidazolines conformément à la formule générale I, dans laquelle $R^7$ est l'hydrogène, et $R^1$, $R^2$, $R^3$, $R^4$ et $R^8$ ont la signification donnée, caractérisé en ce que l'on fait réagir des composés de formule générale II

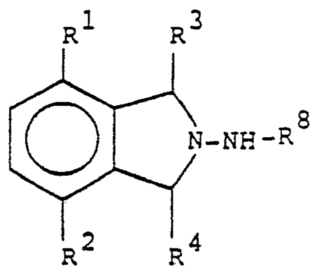

(II)

avec la signification des restes $R^1$ à $R^4$ et $R^8$ donnée, avec un composé de formule générale IV

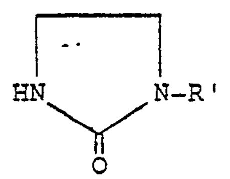

(IV)

dans laquelle R' représente un groupe acyle de 2 à 4 atomes de carbone, et qu'on élimine le groupe acyle.

8. Procédé de préparation d'isoindolinyl-guanidines conformément à la formule général I, dans laquelle les restes $R^1$ à $R^8$ ont la signification donnée, caractérisé en ce que l'on fait réagir des composés de formule générale II

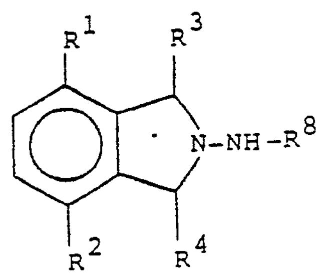

(II)

avec la signification de $R^1$ à $R^4$ et $R^8$ donnée ci-dessus, avec du cyanamide ou avec des cyanamides correspondants substitués par $R^5$, $R^6$ et $R^7$.

9. Procédé de préparation d'isoindolinyl-guanidines conformément à la formule générale I, dans laquelle les restes $R^1$ à $R^8$ ont la signification donnée, caractérisé en ce que l'on fait réagir des composés de formule générale II

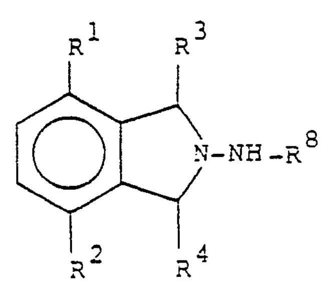

(II)

dans laquelle $R^1$, $R^2$, $R^3$, $R^4$ et $R^8$ ont la signification donnée ci-dessus, avec des isothiocyanates aliphatiques de formule $R^5NCS$, avec la signification de $R^5$ donnée ci-dessus, en thiosemicarbazides de

formule générale V,

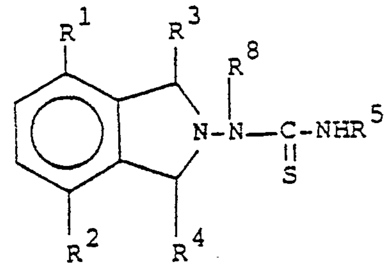

(V)

dans laquelle $R^1$, $R^2$, $R^3$, $R^4$, $R^5$ et $R^8$ ont la signification donnée ci-dessus, en ce que l'on soumet ces thiosemicarbazides à une S-méthylation avec de l'iodure de méthyle et que l'on fait réagir finalement les produits de réaction retenus avec de l'ammoniac glacé, ou des amines primaires ou secondaires.

10. Procédé de préparation d'isoindolinyl-guanidines conformément à la formule générale I, dans laquelle les restes $R^1$, $R^2$, $R^3$, $R^4$, $R^6$, $R^7$ et $R^8$ ont la signification donnée et $R^5$ est l'hydrogène, caractérisé en ce que l'on fait réagir des composés de formule II

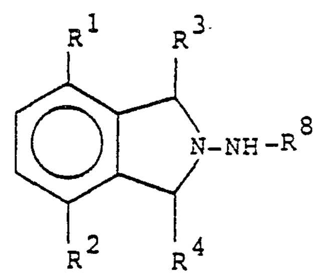

(II)

avec la signification des restes $R^1$ à $R^4$ et $R^8$ donnée ci-dessus avec de l'isothiocyanate de benzoyle en thiosemicarbazides de formule générale VI,

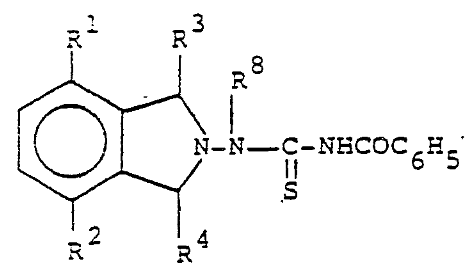

(VI)

qui sont transformés en les composés de formule générale VII

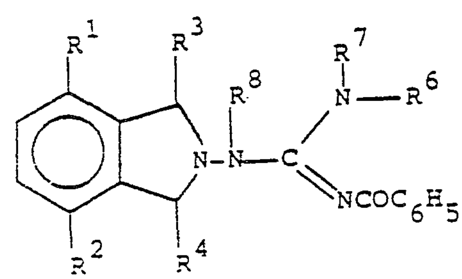

(VII)

par réaction avec des amines de formule générale $R^6R^7NH$ avec la signification de $R^6$ et $R^7$ donnée ci-dessus, et en ce que l'on élimine finalement le groupe benzoyle.

11. Procédé de préparation d'isoindolinyl-guanidines et -imidazolines conformément à la formule générale I avec la signification des restes $R^1$ à $R^8$ donnée alors, caractérisé en ce que l'on fait réagir des composés de formule générale VI

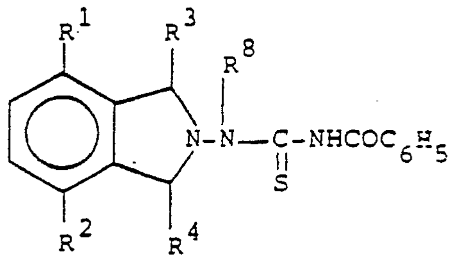

(VI)

avec la signification des restes $R^1$ à $R^4$ et $R^8$ donnée ci-dessus, par élimination du reste benzoyle en les thiourées correspondantes de formule VIII,

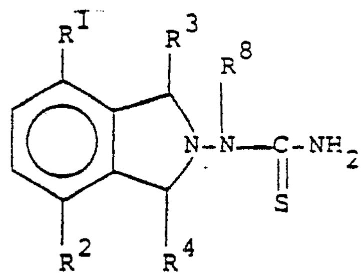

(VIII)

qui sont transformées en les sels S-alkylisothiouronium, et en ce que l'on fait réagir ces composés avec des amines primaires ou secondaires en guanidines ou avec du diamino-1,2 éthane en imidazolines.

12. Préparation pharmaceutique caractérisée en ce qu'elle contient l'un ou plusieurs des composés selon la revendication 1 ou des sels de ceux-ci physiologiquement acceptables et le cas échéant des excipients usuels et/ou un diluant.

13. Composés de formule générale I ou leurs sels obtenus par addition d'acide, physiologiquement acceptables, selon la revendication 1 destinés à l'administration lors des états hypotoniques.

14. Composés de formule générale I ou leurs sels obtenus par addition d'acide, physiologiquement acceptables, destinés à l'administration en rhinologie.

15. (Dichloro-4,7-isoindolin-2-yl)-guanidine.

16. Dichloro-4,7-(imidazolin-2-ylamino-2)-2-isoindoline.

17. Isoindolinyl-aminoimidazolines et isoindolinylguanidines selon la revendication 1, conformément à la formule générale I dans laquelle $R^8$ représente le groupe méthyle et les substituants restants ont la signification donnée.

**Revendications** (pour l'Etat contractant AT)

1. Procédé de préparation d'isoindolin-2-yl-guanidines et -aminoimidazolines de formule générale I

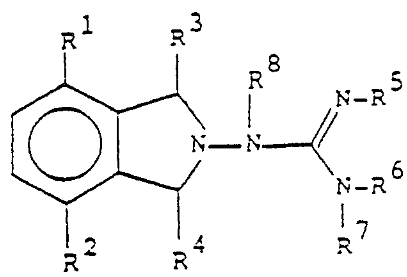

(I)

dans laquelle

$R^1$ et $R^2$ qui peuvent être identiques ou différents, représentent l'hydrogène ou un halogène, dans lequel au moins l'un des substituants $R^1$, $R^2$ est un halogène,

$R^3$ et $R^4$ qui peuvent être identiques ou différents, représentent l'hydrogène ou un alkyle de 1 à 4 atomes de carbone,

$R^5$, $R^6$, $R^7$ et $R^8$ qui peuvent être identiques ou différents, représentent l'hydrogène ou un alkyle de 1 à 4 atomes de carbone ou

$R^5$ et $R^6$ forment ensemble le groupe éthylène,

ainsi que leurs sels, à l'exception de la chloro-4-(imidazoline-2-ylamino-2)-2-isoindoline, caractérisé en ce que l'on fait réagir un composé de formule II

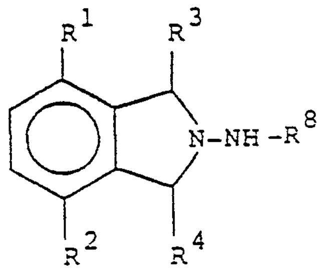

(II)

avec la signification de $R^1$, $R^2$, $R^3$, $R^4$ et $R^8$ donnée ci-dessus, avec un composé de formule générale III

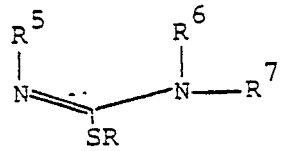

(III)

avec la signification de $R^5$, $R^6$ et $R^7$ donnée ci-dessus, dans laquelle R représente un groupe alkyle, et en ce que l'on convertit, si désiré, les bases libres avec des acides en les sels correspondants obtenus par addition d'acide et

a) pour la préparation d'isoindolinylimidazolines conformément à la formule générale I, dans laquelle $R^7$ est l'hydrogène et $R^1$, $R^2$, $R^3$, $R^4$ et $R^8$ ont la signification donnée, on fait réagir des composés de formule générale II

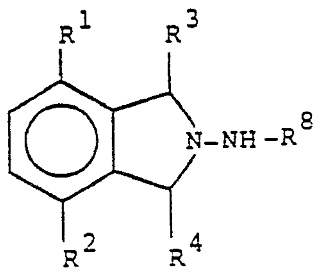

(II)

avec la signification des restes $R^1$ à $R^4$ et $R^8$ donnée, avec un composé de formule générale IV

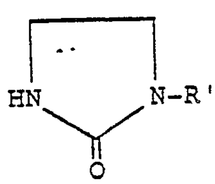

(IV)

dans laquelle R' représente un groupe acyle de 2 à 4 atomes de carbone, et l'on élimine le groupe acyle et l'on convertit, si désiré, les bases libres avec des acides en les sels correspondants obtenus par addition d'acide et

b) pour la préparation d'isoindolinyl-guanidines conformément à la formule générale I, dans laquelle les restes $R^1$ à $R^8$ ont la signification donnée, on fait réagir des composés de formule générale II

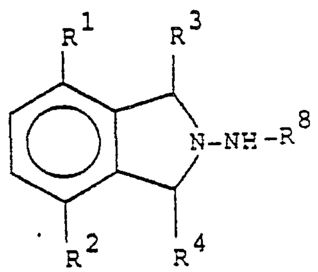

(II)

avec la signification de $R^1$ à $R^4$ et $R^8$ donnée ci-dessus, avec du cyanamide ou avec des cyanamides

correspondants substitués par $R^5$, $R^6$ et $R^7$, et l'on convertit, si désiré, les bases libres avec des acides en les sels correspondants obtenus par addition d'acide et

c) pour la préparation d'isoindolinyl-guanidines conformément à la formule générale I, dans laquelle les restes $R^1$ à $R^8$ ont la signification donnée, on fait réagir des composés de formule générale II

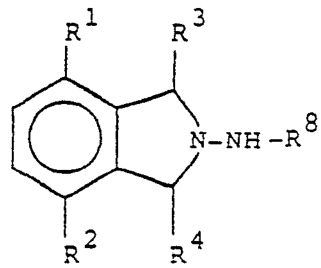

(II)

dans laquelle $R^1$, $R^2$, $R^3$, $R^4$ et $R^8$ ont la signification donnée ci-dessus, avec des isothiocyanates aliphatiques de formule $R^5NCS$ avec la signification de $R^5$ donnée ci-dessus, en thiosemicarbazides de formule générale V

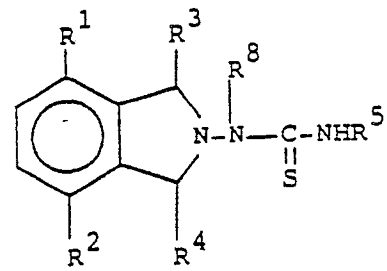

(V)

dans laquelle $R^1$, $R^2$, $R^3$, $R^4$, $R^5$ et $R^8$ ont la signification donnée ci-dessus, l'on soumet ces thiosemicarbazides à une S-méthylation à l'aide d'iodure de méthyle et l'on fait réagir finalement les produits de réaction obtenus avec de l'ammoniac ou des amines primaires ou secondaires substituées par $R^6$ et $R^7$, et l'on convertit, si désiré, les bases libres avec des acides en les sels correspondants obtenus par addition d'acide et

d) pour la préparation d'isoindolinyl-guanidines conformément à la formule générale I, dans laquelle les restes $R^1$, $R^2$, $R^3$, $R^4$, $R^6$, $R^7$ et $R^8$ ont la signification donnée et $R^5$ est l'hydrogène, l'on fait réagir des composés de formule générale II

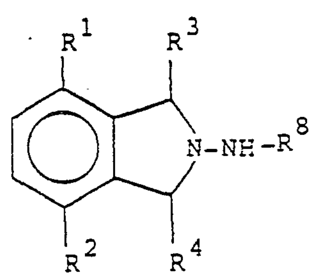

(II)

avec la signification des restes $R^1$ à $R^4$ et $R^8$ donnée ci-dessus avec de l'isothiocyanate de benzoyle en thiosemicarbazides de formule générale VI

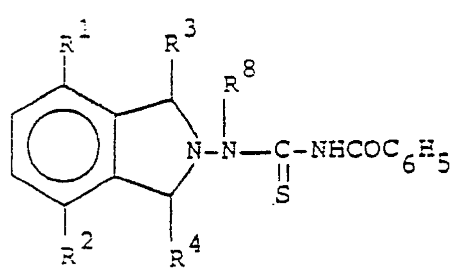

(VI)

qui sont transformés en les composés de formule générale VII

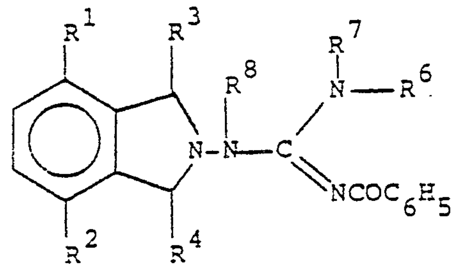

(VII)

par réaction avec des amines de formule générale $R^6R^7NH$, avec la signification de $R^6$ et $R^7$ donnée ci-dessus, et l'on élimine finalement le groupe benzoyle et l'on convertit, si désiré, les bases libres avec des acides en les sels correspondants obtenus par addition d'acide et

e) pour la préparation d'isoindolinyl-guanidines et -imidazolines conformément à la formule générale I avec la signification des restes $R^1$ à $R^8$ donnée alors, l'on convertit des composés de formule générale VI

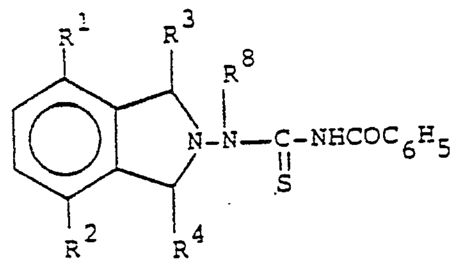

(VI)

avec la signification des restes $R^1$ à $R^4$ et $R^8$ donnée ci-dessus, par élimination du reste benzoyle en les thiourées correspondantes de formule VIII,

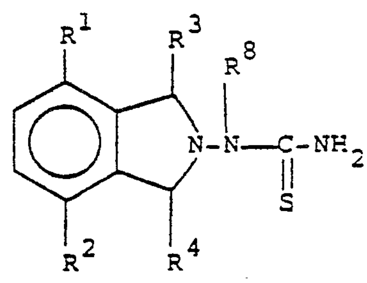

(VIII)

qui sont transformées par alkylation en les sels de S-alkylisothiouronium, et en ce que l'on convertit ces composés en guanidines soit par des amines primaires ou secondaires substituées par $R^6$ et $R^7$ ou bien en imidazolines avec du diamino-1,2 éthane et l'on convertit, si désiré, les bases libres avec des acides en les sels correspondants obtenus par addition d'acide.

2. Procédé de préparation de (dichloro-4,7-isoindolin-2-yl)-guanidine selon la revendication 1, caractérisé en ce que l'on fait réagir la dichloro-4,7-isoindoline avec du cyanamide.

3. Procédé de préparation de médicaments, caractérisé en ce que l'on formule un ou plusieurs composés de formule générale I ou leurs sels obtenus par addition d'acide, physiologiquement acceptables, avec des adjuvants, des excipients ou des lubrifiants galéniques usuels, comme par exemple des substances pour obtenir une action retard chez des comprimés, des capsules ou des solutions.